# EUROPEAN PATENT SPECIFICATION

(11) **EP 2 396 776 B1**
(45) Date of publication and mention of the grant of the patent: **13.06.2012**
(21) Application number: 10705185.6
(22) Date of filing: 08.02.2010
(51) Int. Cl.: G08B 21/22, A61B 5/00, G08B 21/04

(54) **BED MONITORING SYSTEM**
BETTÜBERWACHUNGSSYSTEM
SYSTÈME DE CONTRÔLE D'UN LIT

(30) Priority: 13.02.2009 EP 09152809
(43) Date of publication of application: 21.12.2011
(73) Proprietor: Koninklijke Philips Electronics N.V., 5621 BA Eindhoven (NL); Philips Intellectual Property & Standards GmbH, 20099 Hamburg (DE)
(72) Inventor: STADLTHANNER, Kurt, NL-5656 AE Eindhoven (NL); BRAUERS, Andreas, NL-5656 AE Eindhoven (NL)
(74) Representative: Kroeze, Johannes Antonius
(86) International application number: PCT/IB2010/050548
(87) International publication number: WO 2010/092517

(56) References cited:
- EP-A1- 1 639 941
- US-A1- 2006 042 409
- US-A1- 2008 039 736

## Description

### TECHNICAL FIELD OF THE INVENTION

The invention relates to a bed monitoring system and method, and in particular relates to a bed monitoring system and method that monitors the pressure or force exerted on the bed to determine the state of a user relative to the bed.

### BACKGROUND TO THE INVENTION

It is important in several applications to monitor whether a user or patient is in their bed, or whether the user or patient has got up and left their bed. The most prominent applications are the care of the elderly, care of children and care of patients with specific conditions or circumstances, for example post-surgery.

There are several prior art devices available that can detect when a patient has left the bed. Usually, these devices use either pressure switches or pressure sensing mats to measure the pressure or load on the bed. In general, an alarm is triggered when the measured pressure falls below a predefined threshold. Document US 20060042409 discloses monitoring biosignals to determine whether a patient is in or out of bed.

Usually, carers are interested in two events when a bed monitoring system is being used. Firstly, they want to be alerted when the patient or user starts to leave the bed, and secondly, the alert should be cancelled once the user or patient is safely in bed again (i.e. they are laying down in bed and have come to rest).

Between these two events, the monitored user or patient may return to bed, but, for example, only sit on the edge of the bed, or may lie down but remain very active. Both situations often precede the user or patient getting out of bed again, so it is desirable for the alarm to remain active in these cases. Likewise, there should be no spurious activations or cancellations of the alarm when someone makes up the bed, or undertakes similar actions.

Therefore, it is an object of the invention to provide a bed monitoring system and method that can provide an indication of the state of the user relative to the bed, and in which this indication can be used to improve the reliability of the activation and deactivation of the alarm condition.

### SUMMARY OF THE INVENTION

Therefore, according to a first aspect of the invention, there is provided a bed monitoring system comprising a sensor for detecting and measuring the pressure or force exerted on a bed; and a processor configured to process signals from the sensor to identify a pressure or force pattern that is characteristic of a state of a user relative to the bed; wherein the pressure or force pattern is identified by examining the measured pressure or force over time.

According to a second aspect of the invention, there is provided a method of monitoring a bed, the method comprising detecting and measuring the pressure or force exerted on a bed using a sensor; and processing signals from the sensor to identify a pressure or force pattern that is characteristic of a state of a user relative to the bed; the pressure or force pattern being identified by examining the measured pressure or force over time.

According to a third aspect of the invention, there is provided a computer program product, comprising computer program code that, when executed on a computer or processor, is configured to process signals from a pressure or force sensor to identify a pressure or force pattern that is characteristic of a state of a user relative to a bed, the pressure or force pattern being identified by examining the measured pressure or force over time.

### BRIEF DESCRIPTION OF THE DRAWINGS

Various embodiments of the invention will now be described, by way of example only, with reference to the following drawings, in which:
Fig. 1 is an illustration of a bed monitoring system in accordance with the invention;
Fig. 2 is a block diagram of a bed monitoring system in accordance with the invention;
Fig. 3 shows plots of the signal amplitude, autocorrelation coefficients and variance for an exemplary set of samples from a sensor; and
Fig. 4 is a flow chart illustrating a method in accordance with the invention.

### DETAILED DESCRIPTION OF THE PREFERRED EMBODIMENTS

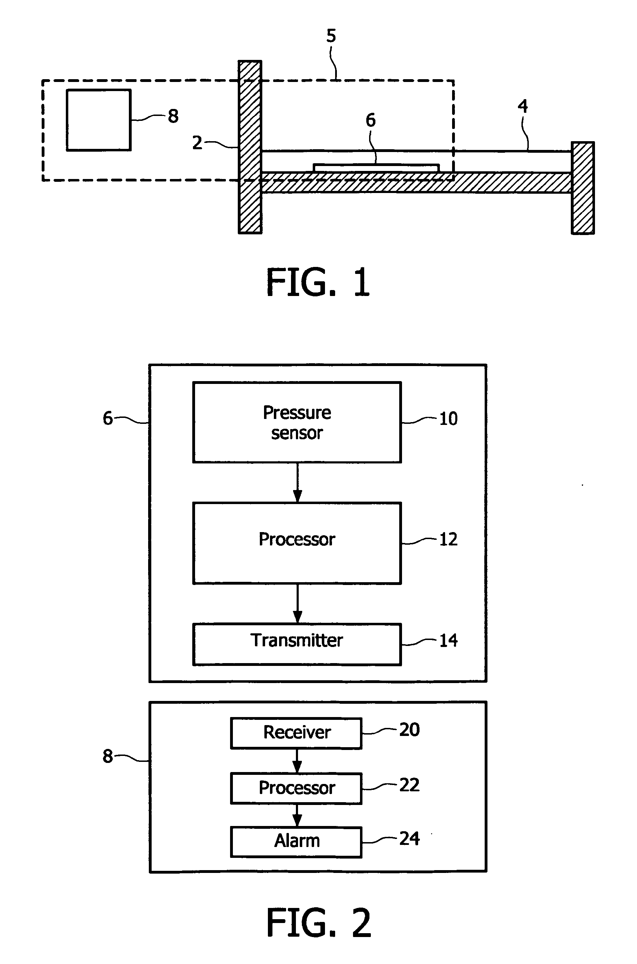

In Fig. 1, a bed 2 is shown that has a mattress 4. A bed monitoring system 5 is provided for monitoring the state of a user (not shown) relative to the bed 2. In this illustrated embodiment, the bed monitoring system 5 comprises a sensor unit 6 that is provided beneath the mattress 4, but in alternative embodiments, the sensor unit 6 may be provided on top of the mattress 4, or as an integral part of the bed 2, or integrated into the mattress 4.

The bed monitoring system 5 also comprises a base station 8, which can be located near to the bed 2 or further away at a monitoring station (such as a nurse's station, for example), that communicates with the sensor unit 6, and which can sound an alarm or warning that a person on the bed 2 is getting out of the bed 2, or has got out of the bed 2.

Fig. 2 shows the bed monitoring system 5 in more detail. The sensor unit 6 comprises a sensor 10 that generates signals indicating the pressure or forces exerted on the bed 2. A processor 12 is provided that receives the signals from the sensor 10 and processes the signals to determine the state of the user relative to the bed 2.

The sensor unit 6 can comprise a single sensor 10 that is placed in a generally central position on or in the bed 2 or on or in the mattress 4, or in a position on the bed 2 or mattress 4 that corresponds to the position of the user when they are lying correctly on the bed 2. Alternatively, the sensor unit 6 can comprise a number of sensors 10 that are distributed over or in the bed 2 so that the pressure or force can be measured at different points.

The sensor 10 can be a force sensor for measuring the forces exerted on the bed 2 by the user, a pressure sensor for measuring the pressure exerted on the bed 2 by the user, or a strain gauge for measuring the strain on the bed 2 caused by the user. In this latter embodiment, the measurements from the strain gauge are used to determine the pressure being exerted on the bed 2 by the user. In preferred embodiments, the sensor 10 is selected from a piezoelectric switch, a piezoelectric foil, an electret foil or a strain gauge. Those skilled in the art will appreciate that alternative types of sensors can be used to perform the method in accordance with the invention.

It will be appreciated that in the embodiments in which the sensor 10 is placed under the mattress 4, some component of the measured signal will represent the weight of the mattress 4 (and any bedding) acting on the bed 2. In accordance with embodiments of the invention discussed further below, the algorithm used to analyze the measurements from the sensor 10 can be adapted to compensate for these components.

As described in more detail below, the bed monitoring system 5 can determine a number of different states of the user relative to the bed 2 by identifying corresponding pressure or force patterns from changes in the measured pressure or forces over time.

The different "states" of the user relative to the bed 2 distinguishable by the bed monitoring system 5 according to the invention can include one or more of the following: the user lying on the bed 2 without substantial movement that is indicative of the user getting out of bed, the user lying on the bed 2 but being quite active or restless, the user starting to get out of the bed 2, the user sitting on the edge of the bed 2, the bed 2 being empty and the bed being manipulated in some way (for example the bed 2 is being made up).

If the processor 12 does determine that the user is likely to get out of bed 2 (i.e. the user is lying on the bed 2 but is quite active or restless or is sitting on the edge of the bed 2), the user is getting out of bed 2 or if the bed 2 is empty, a warning or alert signal can be provided to transmitter 14 that transmits the warning or alarm signal to the base station 8.

In some embodiments, the warning or alarm signal can include information identifying the particular state of the user relative to the bed 2.

The base station 8 comprises a receiver 20 for receiving the warning or alarm signal from the sensor unit 6, a processor 22 and an alarm 24 for issuing an alarm or warning that the user is about to get out of the bed 2, or has got out of the bed 2.

In further embodiments, the processor 12 can provide a signal to the transmitter 14 indicating that the user is lying on the bed 2 without substantial movement (i.e. the user is lying on the bed 2 asleep or without any indication that they are going to get out of the bed 2). In this case, the base station 8 can issue an indication that the user is on the bed 2.

It will be appreciated that, in alternative embodiments, the processing of the signals from the sensor 10 to determine the state of the user relative to the bed 2 can be performed by the processor 22 in the base station 8 rather than the processor 12 in sensor unit 6, in which case the sensor unit 6 transmits the signals from the sensors 10 to the base station 8 for analysis.

It will be further appreciated that the functions of the sensor unit 6 and base station 8 can be integrated into a single device.

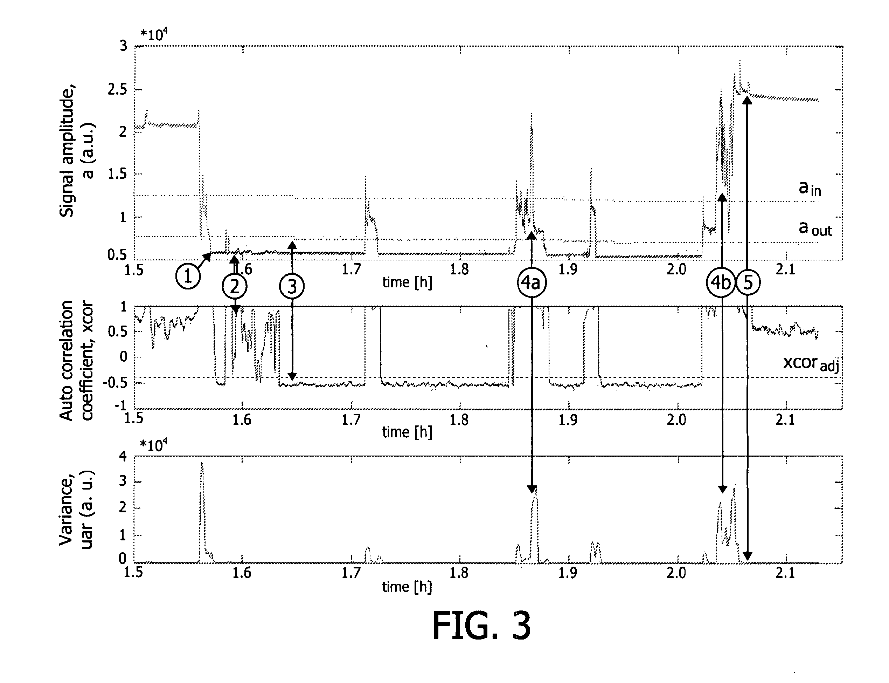

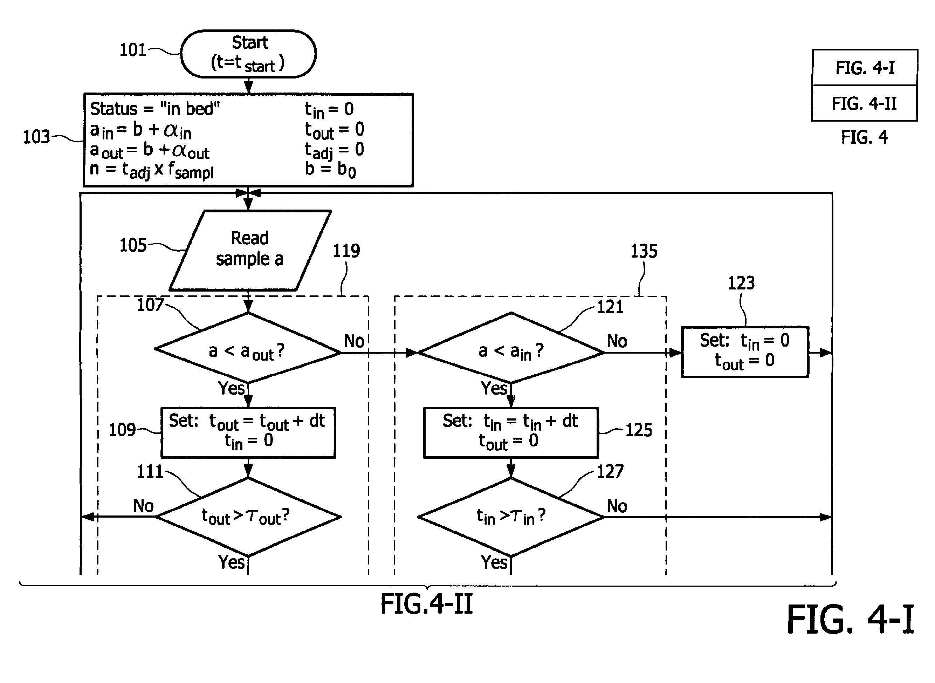

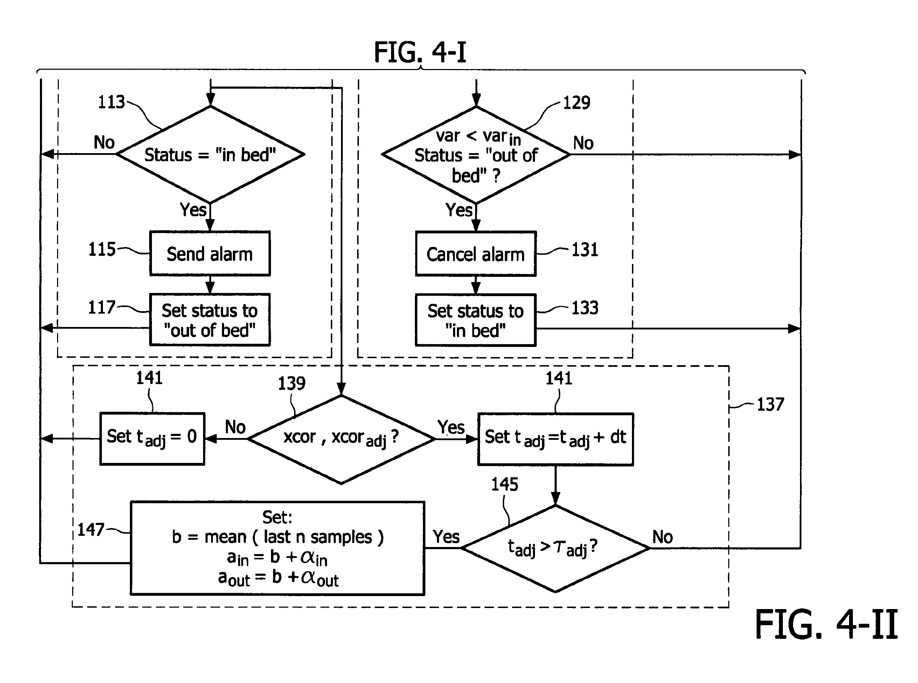

The operation of the invention will now be illustrated with reference to Fig. 3 and 4.

Fig. 3(a) shows a typical signal obtained from a single pressure or force sensor 10 covering a period of time in which the user and bed 2 are in several different states relative to each other. Two threshold values for the signal amplitude, aᵢₙ and aₒᵤₜ, are indicated in Fig. 3(a). As described further below, in preferred embodiments of the invention, the threshold values can be dynamically set during the operation of the bed monitoring system 5 in order to adapt to changes in the ambient conditions and/or in the event that the signals from the sensor 10 show drift.

Fig. 3(b) is a plot of the autocorrelation coefficient, xcor, of the signal in Fig. 3(a), which is calculated by the processor 12 (or the processor 22 in the alternative embodiment in which the processing is performed in the base station 8). In particular, a moving window of length d is used, and in each window the autocorrelation coefficient xcor (delayed by one data point) is computed.

Fig. 3(c) is a plot of the variance, var of the signal in Fig. 3(a), which, again, is calculated by the processor 12 (or processor 22 in the alternative embodiment), and in which a moving window of size d is used.

Initially, the bed 2 is occupied by a user that is resting calmly, and this is represented by the first segment, from t = 1.5 to t = 1.57. At the point marked by "1" (at approximately t = 1.57) the user has sat up, but is still on the edge of the bed 2. Since, in this position, the upper body of the user hardly imposes any load onto the pressure sensor 10, the signal amplitude falls below the lower threshold aₒᵤₜ.

While the user is still sitting on the bed 2, some small pressure changes are detected by the sensor 10 (as shown by the point "2"). These small changes are more clearly evident from the corresponding part of the plot shown in Fig. 3(b), which shows that the autocorrelation coefficients at this time have relatively high values.

After the user has eventually left the bed (indicated by point "3"), the value of the autocorrelation coefficients fall and remain below a threshold xcor_{adj} (shown in Fig. 3(b)). Therefore, an empty bed 2 is characterized by both a low signal amplitude (i.e. below threshold aₒᵤₜ) as well as a low autocorrelation coefficient of the signal (i.e. below threshold xcor_{adj}).

Alternatively, in situations in which there is a high signal-to-noise ratio, the autocorrelation criterion could be replaced by considering the variance, as shown in Fig. 3(c). In this case, an empty bed 2 would then be characterized by a low signal amplitude (below threshold aₒᵤₜ) together with a variance smaller than a threshold varᵢₙ (not shown in Fig. 3(c)).

In order to determine whether the user has returned to bed properly (i.e. they are laying down asleep or they are laying down without being active), the amplitude of the signal is analyzed along with the corresponding variance. Once the user has lain down on the bed 2, the signal amplitude will exceed the upper threshold aᵢₙ (as shown by point "4b" in Fig. 3(a)). At this point, the variance of the signal is high since the user is still moving around in bed 2. The variance of the signal falls to a low or negligible value once the user has stopped moving and has settled down (as shown by point "5" in Fig. 3(c)).

At point "4a" in Fig. 3(a), it can be seen that the signal amplitude is briefly above the upper threshold aᵢₙ. However, as the signal amplitude drops below the upper and lower thresholds (aᵢₙ and aₒᵤₜ) a short time later, this is not indicative of the user returning to bed 2, but instead is indicative of the bed 2 being manipulated, such as the bed 2 being made up. Furthermore, the high variance of the signal also indicates that the user has not lain down calmly in the bed 2.

Thus, it can be seen from the above that an evaluation of the amplitude of the signal from the sensor 10 in combination with an evaluation of the autocorrelation coefficient of the signal or the variance of the signal allows various different states of the user and the bed 2 to be determined. In particular, it is possible to distinguish between the states of: the user lying on the bed 2 asleep or at rest, the user lying on the bed 2 but being quite active or restless, the user sitting on the edge of the bed 2, the bed 2 being empty or the bed 2 being manipulated in some way (for example being made up).

Fig. 4 is a flow chart illustrating the processing path of the processor 12 in the sensor unit 6 (or processor 22 in the base station 8 in the alternative embodiment).

The method starts in step 101, at time t = tₛₜₐᵣₜ, and it is assumed that the bed 2 is occupied by the user. For example, an operator of the system (for example a care provider for the user) may only activate the bed monitoring system 5 once the user is in the bed 2. It will be appreciated, however, that the initial assumption can be that the bed 2 is unoccupied. As a further alternative (not shown), part of the initialization process in the method can determine whether the bed 2 is initially occupied or unoccupied.

In step 103, various parameters are initialized. In particular, the status of the bed (in terms of whether the user is in bed or out of bed) is set to "in bed", the upper threshold for the signal amplitude aᵢₙ is set to b + αᵢₙ, where b is value initially set equal to an operator defined value b₀, the lower threshold for the signal amplitude aₒᵤₜ is set to b + αₒᵤₜ, the time, tᵢₙ, that the signal amplitude has exceeded the upper threshold aᵢₙ is set to 0, the time, tₒᵤₜ, that the signal amplitude has been below the lower threshold aₒᵤₜ is set to 0, a further time parameter, t_{adj}, is set to 0, and a number of samples n is set equal to the product of t_{adj} (initially 0) and the sampling frequency, fₛₐₘₚₗ, used by the sensor 10. The parameters αᵢₙ, αₒᵤₜ, τᵢₙ, τₒᵤₜ and xcor_{adj} are predefined and remain constant throughout the operation of the method.

Two threshold values for the signal amplitude are provided in order to avoid, or reduce the risk of, false alarms. The lower threshold, aₒᵤₜ is preferably set to a small value such that the signal amplitude should only fall below this value when the user has sat up or left the bed 2, not when the user is simply lying close to the edge of the bed 2.

The upper threshold, aᵢₙ, is set high enough such that the signal amplitude will not exceed this value if the user is sitting on the edge of the bed 2. The signal amplitude can fall below this value when the user is lying on the edge of the bed 2.

In step 105, the processor 12/22 reads the next sample, a, from the sensor 10, and compares it to the current value of the lower threshold, aₒᵤₜ (step 107).

If the amplitude of the sample a is lower than the lower threshold, aₒᵤₜ, the method proceeds to step 109 in which the value of tₒᵤₜ is increased by an amount dt, where dt = 1/fₛₐₘₚₗ, and the value of tᵢₙ is set to 0 (or reset if tᵢₙ was previously non-zero).

Next, in step 111, the updated value of tₒᵤₜ is compared to a threshold value τₒᵤₜ. If the value of tₒᵤₜ is less than the threshold value τₒᵤₜ, the method returns to step 105 and the next sample a is read and processed.

If the value of tₒᵤₜ is greater than the threshold value τₒᵤₜ, the method moves to step 113. In step 113, it is determined whether the Status is "in bed".

If the Status is "in bed", the method moves to step 115 in which an alarm signal is sent to the base station 8 of the system 5. If the Status is not "in bed" (i.e. the status is "out of bed", which means that the alarm has already been raised), the method returns to step 105 and the next sample a is read and processed.

After step 115, the method passes to step 117 in which the Status is set to "out of bed", and the method returns to step 105 and the next sample a is read and processed.

Thus, this branch of the overall method, as indicated by dashed box 119, is concerned with detecting when the user has got out of the bed 2. In particular, the effect of step 111 and the value of threshold τₒᵤₜ is to ensure that the alarm is not activated inadvertently when the user is just moving around or turning over on the bed 2 (which can result in the amplitude of the signal temporarily dropping below the lower threshold aₒᵤₜ). Thus, the alarm condition is only activated when the signal amplitude falls below the threshold aₒᵤₜ for a period of time greater than τₒᵤₜ.

Returning to step 107, if the signal amplitude is greater than the lower threshold aₒᵤₜ, the method passes to step 121 in which the signal amplitude a is compared to the upper threshold, aᵢₙ.

If the signal amplitude is below the upper threshold, aᵢₙ (i.e. the signal amplitude is between the upper and lower threshold values following from step 107), the method passes to step 123 in which tᵢₙ and tₒᵤₜ are set or reset to 0. The method then returns to step 105 and the next sample a is read and processed.

If the signal amplitude is above the upper threshold, aᵢₙ, the method moves to step 125 in which tᵢₙ is incremented by an amount dt, and tₒᵤₜ is set or reset to 0.

Next, in step 127, the updated value of tᵢₙ is compared to a threshold value τᵢₙ. If the value of tᵢₙ is less than the threshold value τᵢₙ, the method returns to step 105 and the next sample a is read and processed.

If the value of tᵢₙ is greater than the threshold value τᵢₙ, the method moves to step 129. In step 129, it is determined whether the variance is below a variance threshold, varᵢₙ and whether the Status is "out of bed".

If one or both of these criteria are not satisfied, the method returns to step 105 and the next sample a is read and processed.

If both of these criteria are satisfied, the method moves to step 131 in which the alarm is cancelled.

In the following step, step 133, the Status is set to "in bed" and then the method returns to step 105 and the next sample a is read and processed.

Thus, this branch of the overall method, as indicated by dashed box 135, is concerned with detecting when the user has got back into the bed 2 and is at rest. In particular, the effect of steps 127 and 129 is to ensure that the alarm is only deactivated when the user has properly got back into bed 2, and that the alarm is not deactivated when the bed 2 is being manipulated in some way (for example the bed 2 is being made up). Thus, the alarm condition is only deactivated once the signal amplitude has been above the threshold aᵢₙ for a period of time greater than τᵢₙ and the variance is below the variance threshold varᵢₙ.

In an embodiment of the invention, the method can further comprise the steps shown in dashed box 137, which relate to the dynamic adjustment of the thresholds aᵢₙ and aₒᵤₜ. As described above, this adjustment can be used to make sure that the measurements from the sensor 10 are calibrated and interpreted correctly, and to counteract drift in the measurements. Thus, it is necessary to determine when the bed 2 is empty. Therefore, after a positive response to the decision in step 111, the value of the autocorrelation coefficient is compared with a threshold value, xcor_{adj}.

If the autocorrelation coefficient xcor is greater than the threshold value, the method moves to step 141 in which the time t_{adj} is set or reset to 0. The method then returns to step 105 and the next sample a is read and processed.

If the autocorrelation coefficient xcor is less than the threshold value, the method moves to step 143 in which the time t_{adj} is incremented by an amount dt.

The incremented time is compared with a threshold time value, τ_{adj}, in step 145. If the incremented time t_{adj} is less than the threshold value τ_{adj} then the method returns to step 105 and the next sample a is read and processed.

If the incremented time t_{adj} is greater than the threshold value τ_{adj} then the method passes to step 147 in which the value of the sensor baseline parameter b is set to be equal to the mean of the last n samples. The values of the upper threshold aᵢₙ and lower threshold aₒᵤₜ are adjusted based on the new value for b.

The method then returns to step 105 and the next sample a is read and processed.

In the exemplary situation shown in Fig. 3, the upper and lower thresholds will only be adapted if both the amplitude of the signal is lower than aₒᵤₜ and if the autocorrelation criterion is met. In this way, it should be possible to avoid the situation in which the thresholds are modified when a heavy object is placed onto the bed. It is possible, in further embodiments, for the bed monitoring system to provide an indication to the care provider about abnormal changes in the baseline b by issuing a notification once the signal amplitude is larger than aₒᵤₜ and the autocorrelation criterion is met.

In further embodiments of the invention, the operation of the bed monitoring system 5 can be enhanced by providing further sensors to detect vital signs of the user (such as heart rate, heart rate variability or breathing rate). The measurements that these additional sensors provide can be used to reduce the time taken to confirm that a bed departure event has occurred, and can be used to improve the determination of whether the user is resting in bed, or asleep in bed. This method has been described in European patent application number EP08164045.0, filed on 10 September 2008 in the name of Koninklijke Philips Electronics N.V., the content of which is hereby incorporated by reference.

There is therefore provided a bed monitoring system and method that can provide an indication of the state of the user relative to the bed, and in which this indication can be used to improve the reliability of the activation and deactivation of the alarm condition.

While the invention has been illustrated and described in detail in the drawings and foregoing description, such illustration and description are to be considered illustrative or exemplary and not restrictive; the invention is not limited to the disclosed embodiments.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A bed monitoring system, comprising:
- a sensor for detecting and measuring the pressure or force exerted on a bed; and
- a processor configured to process signals from the sensor to identify a pressure or force pattern that is characteristic of a state of a user relative to the bed, wherein the processor is configured to examine the amplitude and variance of the signals from the sensor to identify a pressure or force pattern that is characteristic of a state of a user relative to the bed;
wherein the pressure or force pattern is identified by examining the measured pressure or force over time;
**characterized in that**
the processor is configured to determine that the user is in bed and lying restfully in the event that the amplitude of the signals from the sensor is above a first amplitude threshold for more than a predetermined period of time and the variance of the signals is below a variance threshold during said predetermined period of time.

2. A bed monitoring system as claimed in claim 1, wherein the processor is configured to determine that the user has got or is getting out of the bed in the event that the amplitude of the signals from the sensor is below a second amplitude threshold for more than a predetermined period of time.

3. A bed monitoring system as claimed in claim 2, wherein in the event that the processor determines that the user has got or is getting out of the bed, the processor is configured to generate an alarm or warning signal.

4. A bed monitoring system as claimed in claim 1, 2 or 3, wherein the processor is further configured to examine an autocorrelation coefficient of the signals from the sensor.

5. A bed monitoring system as claimed in claim 4, wherein the processor is configured to determine that the user is sat on the edge of the bed in the event that the amplitude of the signals from the sensor is below a or the second amplitude threshold and the autocorrelation coefficient is above an autocorrelation coefficient threshold.

6. A bed monitoring system as claimed in claim 1, wherein the processor is configured to determine that the bed is being manipulated and/or the user is in bed but restless in the event that the amplitude of the signals from the sensor is above the first amplitude threshold and the variance is above the variance threshold.

7. A bed monitoring system as claimed in claim 1, wherein in the event that the processor determines that the user is in bed and lying restfully, the processor is configured to cancel an alarm or warning signal.

8. A bed monitoring system as claimed in claim 4 or 5, wherein the processor is configured to determine that the user is out of the bed and the bed is empty in the event that the amplitude of the signals from the sensor is below a or the second amplitude threshold for more than a predetermined period of time and the autocorrelation coefficient is below an or the autocorrelation coefficient threshold.

9. A bed monitoring system as claimed in claim 1, 6 or 7, wherein the processor is configured to determine that the user is out of the bed and the bed is empty in the event that the amplitude of the signals from the sensor is below a or the second amplitude threshold for more than a predetermined period of time and a variance of the signals from the sensor is below the variance threshold.

10. A bed monitoring system as claimed in claim 8 or 9, wherein when the processor determines that the user is out of the bed and the bed is empty, the processor is further configured to adjust the values of the first and second amplitude thresholds.

11. A bed monitoring system as claimed in claim 8, 9 or 10, when dependent on claim 6, wherein when the processor determines that the user is out of the bed and the bed is empty, the processor is further configured to adjust the value of the first amplitude threshold.

12. A bed monitoring system as claimed in claim 10 or 11, wherein the processor is configured to adjust the value of the first amplitude threshold by setting a parameter on which the first amplitude threshold is based equal to the mean of the amplitude of a plurality of signals from the sensor.

13. A method of monitoring a bed, the method comprising:
- detecting and measuring the pressure or force exerted on a bed using a sensor; and
- processing signals from the sensor to identify a pressure or force pattern that is characteristic of a state of a user relative to the bed; the pressure or force pattern being identified by examining the measured pressure or force over time, wherein the processing comprises examining the amplitude and variance of the signals from the sensor to identify a pressure or force pattern that is characteristic of a state of a user relative to the bed
**characterized in that** the step of processing signals comprises determining that the user is in bed and lying restfully in the event that the amplitude of the signals from the sensor is above an amplitude threshold for more than a predetermined period of time and the variance of the signals is below a variance threshold during said predetermined period of time.

14. A computer program product, comprising computer program code that, when executed on a computer or processor, is configured to:
- process signals from a pressure or force sensor to identify a pressure or force pattern that is characteristic of a state of a user relative to a bed, the pressure or force pattern being identified by examining the measured pressure or force over time, wherein the processing comprises examining the amplitude and variance of the signals from the sensor to identify a pressure or force pattern that is characteristic of a state of a user relative to the bed,
**characterized in that** the computer program code is configured to determine that the user is in bed and lying restfully in the event that the amplitude of the signals from the sensor is above an amplitude threshold for more than a predetermined period of time and the variance of the signals is below a variance threshold during said predetermined period of time.

## Patentansprüche

1. Bettüberwachungssystem mit:
- einem Sensor zum Detektieren und Messen des auf ein Bett ausgeübten Druckes oder der ausgeübten Kraft,
- einem Prozessor, der so konfiguriert ist, dass er von dem Sensor kommende Signale verarbeitet, um ein Druck- oder Kraftmuster zu identifizieren, das kennzeichnend für einen Zustand eines Benutzers hinsichtlich des Bettes ist, wobei der Prozessor derart konfiguriert ist, dass er die Amplitude und die Varianz der vom Sensor kommenden Signale untersucht, um ein Druck- oder Kraftmuster zu identifizieren, das kennzeichnend für einen Zustand eines Benutzers hinsichtlich des Bettes ist,
wobei das Druck- oder Kraftmuster identifiziert wird, indem der gemessene Druck oder die gemessene Kraft im Verlauf der Zeit untersucht wird,
**dadurch gekennzeichnet, dass**
der Prozessor derart konfiguriert ist, dass er ermittelt, dass sich der Benutzer im Bett befindet und ruhig liegt, falls die Amplitude der vom Sensor kommenden Signale länger als eine vorbestimmte Zeitspanne über einem ersten Amplitudenschwellenwert liegt und die Varianz der Signale während der genannten vorbestimmten Zeitspanne unter einem Varianzschwellenwert liegt.

2. Bettüberwachungssystem nach Anspruch 1, wobei der Prozessor so konfiguriert ist, dass er ermittelt, dass der Benutzer aus dem Bett aufgestanden ist oder aufsteht, falls die Amplitude der vom Sensor kommenden Signale länger als eine vorbestimmte Zeitspanne unter einem zweiten Amplitudenschwellenwert liegt.

3. Bettüberwachungssystem nach Anspruch 2, wobei in dem Fall, dass der Prozessor ermittelt, dass der Benutzer aus dem Bett aufgestanden ist oder aufsteht, der Prozessor derart konfiguriert ist, dass er ein Alarm- oder Warnsignal erzeugt.

4. Bettüberwachungssystem nach Anspruch 1, 2 oder 3, wobei der Prozessor ferner so konfiguriert ist, dass er einen Autokorrelationskoeffizienten der vom Sensor kommenden Signale untersucht.

5. Bettüberwachungssystem nach Anspruch 4, wobei der Prozessor so konfiguriert ist, dass er ermittelt, dass der Benutzer auf dem Rand des Bettes sitzt, falls die Amplitude der vom Sensor kommenden Signale unter einem oder dem zweiten Amplitudenschwellenwert liegt und der Autokorrelationskoeffizient über einem Autokorrelationskoeffizienten-Schwellenwert liegt.

6. Bettüberwachungssystem nach Anspruch 1, wobei der Prozessor so konfiguriert ist, dass er ermittelt, dass das Bett manipuliert wird und/oder sich der Benutzer im Bett befindet, aber unruhig ist, falls die Amplitude der vom Sensor kommenden Signale über dem ersten Amplitudenschwellenwert liegt und die Varianz über dem Varianzschwellenwert liegt.

7. Bettüberwachungssystem nach Anspruch 1, wobei in dem Fall, dass der Prozessor ermittelt, dass sich der Benutzer im Bett befindet und ruhig liegt, der Prozessor derart konfiguriert ist, dass er ein Alarm- oder Warnsignal aufhebt.

8. Bettüberwachungssystem nach Anspruch 4 oder 5, wobei der Prozessor so konfiguriert ist, dass er ermittelt, dass sich der Benutzer nicht im Bett befindet und das Bett leer ist, falls die Amplitude der vom Sensor kommenden Signale länger als eine vorbestimmte Zeitspanne unter einem oder dem zweiten Amplitudenschwellenwert liegt und der Autokorrelationskoeffizient unter einem oder dem Autokorrelationskoeffizienten-Schwellenwert liegt.

9. Bettüberwachungssystem nach Anspruch 1, 6 oder 7, wobei der Prozessor so konfiguriert ist, dass er ermittelt, dass sich der Benutzer nicht im Bett befindet und das Bett leer ist, falls die Amplitude der vom Sensor kommenden Signale länger als eine vorbestimmte Zeitspanne unter einem oder dem zweiten Amplitudenschwellenwert liegt und eine Varianz der vom Sensor kommenden Signale unter dem Varianzschwellenwert liegt.

10. Bettüberwachungssystem nach Anspruch 8 oder 9, wobei in dem Fall, dass der Prozessor ermittelt, dass sich der Benutzer nicht im Bett befindet und das Bett leer ist, der Prozessor ferner derart konfiguriert ist, dass er die Werte des ersten und des zweiten Amplitudenschwellenwertes anpasst.

11. Bettüberwachungssystem nach Anspruch 8, 9 oder 10 bei Abhängigkeit von Anspruch 6, wobei in dem Fall, dass der Prozessor ermittelt, dass sich der Benutzer nicht im Bett befindet und das Bett leer ist, der Prozessor ferner derart konfiguriert ist, dass er den Wert des ersten Amplitudenschwellenwertes anpasst.

12. Bettüberwachungssystem nach Anspruch 10 oder 11, wobei der Prozessor so konfiguriert ist, dass er den Wert des ersten Amplitudenschwellenwertes anpasst, indem er einen Parameter, auf dem der erste Amplitudenschwellenwert basiert, gleich dem Mittelwert der Amplitude einer Vielzahl von vom Sensor kommenden Signalen einstellt.

13. Verfahren zur Überwachung eines Bettes, wobei das Verfahren Folgendes umfasst:
- Detektieren und Messen des auf ein Bett ausgeübten Druckes oder der ausgeübten Kraft unter Verwendung eines Sensors und
- Verarbeiten von vom Sensor kommenden Signale, um ein Druck- oder Kraftmuster zu identifizieren, das kennzeichnend für einen Zustand eines Benutzers hinsichtlich des Bettes ist, wobei das Druck- oder Kraftmuster identifiziert wird, indem der gemessene Druck oder die gemessene Kraft im Verlauf der Zeit untersucht wird, wobei die Verarbeitung die Untersuchung der Amplitude und der Varianz der vom Sensor kommenden Signale umfasst, um ein Druck- oder Kraftmuster zu identifizieren, das kennzeichnend für einen Zustand eines Benutzers hinsichtlich des Bettes ist,
**dadurch gekennzeichnet, dass** der Schritt der Signalverarbeitung die Ermittlung umfasst, dass sich der Benutzer im Bett befindet und ruhig liegt, falls die Amplitude der vom Sensor kommenden Signale länger als eine vorbestimmte Zeitspanne über einem Amplitudenschwellenwert liegt und die Varianz der Signale während der genannten vorbestimmten Zeitspanne unter einem Varianzschwellenwert liegt.

14. Computerprogrammprodukt, das einen Computerprogrammcode umfasst, der bei Ausführung auf einem Computer oder Prozessor derart konfiguriert ist, dass er
- von einem Druck- oder Kraftsensor kommende Signale verarbeitet, um ein Druck- oder Kraftmuster zu identifizieren, das kennzeichnend für einen Zustand eines Benutzers hinsichtlich des Bettes ist, wobei das Druck- oder Kraftmuster identifiziert wird, indem der gemessene Druck oder die gemessene Kraft im Verlauf der Zeit untersucht wird, wobei die Verarbeitung die Untersuchung der Amplitude und der Varianz der vom Sensor kommenden Signale umfasst, um ein Druck- oder Kraftmuster zu identifizieren, das kennzeichnend für einen Zustand eines Benutzers hinsichtlich des Bettes ist,
**dadurch gekennzeichnet, dass** der Computerprogrammcode derart konfiguriert ist, dass er ermittelt, dass sich der Benutzer im Bett befindet und ruhig liegt, falls die Amplitude der vom Sensor kommenden Signale länger als eine vorbestimmte Zeitspanne über einem Amplitudenschwellenwert liegt und die Varianz der Signale während der genannten vorbestimmten Zeitspanne unter einem Varianzschwellenwert liegt.

## Revendications

1. Système de surveillance de lit, comprenant :
- un capteur permettant de détecter et de mesurer la pression ou la force exercée sur un lit ; et
- un processeur configuré pour traiter des signaux provenant du capteur afin d'identifier un motif de pression ou de force qui est caractéristique d'un état d'un utilisateur par rapport au lit, dans lequel le processeur est configuré pour examiner l'amplitude et la variance des signaux provenant du capteur afin d'identifier un motif de pression ou de force qui est caractéristique d'un état d'un utilisateur par rapport au lit ;
dans lequel le motif de pression ou de force est identifié en examinant la pression ou la force mesurée dans le temps ;
**caractérisé en ce que**
le processeur est configuré pour déterminer que l'utilisateur est au lit et étendu paisiblement dans le cas où l'amplitude des signaux provenant du capteur est au-dessus d'un premier seuil d'amplitude pendant une période plus longue qu'une période prédéterminée et la variance des signaux est en dessous d'un seuil de variance pendant ladite période prédéterminée.

2. Système de surveillance de lit selon la revendication 1, dans lequel le processeur est configuré pour déterminer que l'utilisateur est sorti ou sort du lit dans le cas où l'amplitude des signaux provenant du capteur est en dessous d'un second seuil d'amplitude pendant une période plus longue qu'une période prédéterminée.

3. Système de surveillance de lit selon la revendication 2, dans lequel dans le cas où le processeur détermine que l'utilisateur est sorti ou sort du lit, le processeur est configuré pour générer une alarme ou un signal d'avertissement.

4. Système de surveillance de lit selon la revendication 1, 2 ou 3, dans lequel le processeur est en outre configuré pour examiner un coefficient d'autocorrélation des signaux provenant du capteur.

5. Système de surveillance de lit selon la revendication 4, dans lequel le processeur est configuré pour déterminer que l'utilisateur est assis sur le bord du lit dans le cas où l'amplitude des signaux provenant du capteur est en dessous d'un ou du second seuil d'amplitude et le coefficient d'autocorrélation est au-dessus d'un seuil de coefficient d'autocorrélation.

6. Système de surveillance de lit selon la revendication 1, dans lequel le processeur est configuré pour déterminer que le lit est en cours de manipulation et/ou l'utilisateur est au lit mais agité dans le cas où l'amplitude des signaux provenant du capteur est au-dessus du premier seuil d'amplitude et la variance est au-dessus du seuil de variance.

7. Système de surveillance de lit selon la revendication 1, dans lequel dans le cas où le processeur détermine que l'utilisateur est au lit et étendu paisiblement, le processeur est configuré pour annuler une alarme ou un signal d'avertissement.

8. Système de surveillance de lit selon la revendication 4 ou 5, dans lequel le processeur est configuré pour déterminer que l'utilisateur est hors du lit et que le lit est vide dans le cas où l'amplitude des signaux provenant du capteur est sous un ou le second seuil d'amplitude pendant une période plus longue qu'une période prédéterminée et le coefficient d'autocorrélation est en-dessous d'un ou du seuil de coefficient d'autocorrélation.

9. Système de surveillance de lit selon la revendication 1, 6 ou 7, dans lequel le processeur est configuré pour déterminer que l'utilisateur est hors du lit et que le lit est vide dans le cas où l'amplitude des signaux provenant du capteur est sous un ou le second seuil d'amplitude pendant une période plus longue qu'une période prédéterminée et une variance des signaux provenant du capteur est sous le seuil de variance.

10. Système de surveillance de lit selon la revendication 8 ou 9, dans lequel lorsque le processeur détermine que l'utilisateur est hors du lit et que le lit est vide, le processeur est en outre configuré pour ajuster les valeurs des premier et second seuils d'amplitude.

11. Système de surveillance de lit selon la revendication 8, 9 ou 10, lorsqu'elle dépend de la revendication 6, dans lequel lorsque le processeur détermine que l'utilisateur est hors du lit et que le lit est vide, le processeur est en outre configuré pour ajuster la valeur du premier seuil d'amplitude.

12. Système de surveillance de lit selon la revendication 10 ou 11, dans lequel le processeur est configuré pour ajuster la valeur du premier seuil d'amplitude en établissant un paramètre sur lequel est basé le premier seuil d'amplitude égal à la moyenne de l'amplitude d'une pluralité de signaux provenant du capteur.

13. Procédé de surveillance d'un lit, le procédé comprenant les étapes consistant à :
- détecter et mesurer la pression ou la force exercée sur un lit avec un capteur ; et
- traiter les signaux provenant du capteur afin d'identifier un motif de pression ou de force qui est caractéristique d'un état d'un utilisateur par rapport au lit ; le motif de pression ou de force étant identifié en examinant la pression ou la force mesurée dans le temps, dans lequel le traitement comprend l'examen de l'amplitude et de la variance des signaux provenant du capteur afin d'identifier un motif de pression ou de force qui est caractéristique d'un état d'un utilisateur par rapport au lit ;
**caractérisé en ce que** l'étape de traitement des signaux comprend la détermination que l'utilisateur est au lit et étendu paisiblement dans le cas où l'amplitude des signaux provenant du capteur est au-dessus d'un premier seuil d'amplitude pendant une période plus longue qu'une période prédéterminée et la variance des signaux est sous un seuil de variance pendant ladite période prédéterminée.

14. Produit de programme informatique, comprenant un code de programme informatique qui, lorsqu'il est exécuté sur un ordinateur ou un processeur, est configuré pour :
- traiter des signaux provenant d'un capteur de pression ou de force afin d'identifier un motif de pression ou de force qui est caractéristique d'un état d'un utilisateur par rapport au lit, le motif de pression ou de force étant identifié par l'examen de la pression ou de la forme mesurée dans le temps, dans lequel le traitement comprend l'examen de l'amplitude et de la variance des signaux provenant du capteur afin d'identifier un motif de pression ou de force qui est caractéristique d'un état d'un utilisateur par rapport au lit,
**caractérisé en ce que** le code de programme informatique est configuré pour déterminer que l'utilisateur est au lit et étendu paisiblement dans le cas où l'amplitude des signaux provenant du capteur est au-dessus d'un seuil d'amplitude pendant une période plus longue qu'une période prédéterminée et la variance des signaux est en dessous d'un seuil de variance pendant ladite période prédéterminée.
